# EUROPÄISCHE PATENTSCHRIFT

(11) **EP 2 234 605 B1**
(45) Veröffentlichungstag und Bekanntmachung des Hinweises auf die Patenterteilung: **05.08.2015**
(21) Anmeldenummer: 08871775.6
(22) Anmeldetag: 09.12.2008
(51) Int. Cl.: A61K 9/70, A61K 47/18, A61K 31/433, A61K 31/522, A61K 31/506, A61K 31/496

(54) **TRANSDERMALES THERAPEUTISCHES SYSTEM MIT HARNSTOFF-KOMPONENTE**
TRANSDERMAL THERAPEUTIC SYSTEM HAVING UREA COMPONENTS
SYSTÈME THÉRAPEUTIQUE TRANSDERMIQUE À BASE D'URÉE

(30) Priorität: 30.01.2008 DE 102008006791
(43) Veröffentlichungstag der Anmeldung: 06.10.2010
(73) Patentinhaber: LTS LOHMANN Therapie-Systeme AG, 56626 Andernach (DE)
(72) Erfinder: HORSTMANN, Michael, 56564 Neuwied (DE); HOFFMANN, Gerd, 56566 Neuwied (DE); WIEDERSBERG, Sandra, 06268 Albersroda (DE)
(74) Vertreter: Siemund, Volker
(86) Internationale Anmeldenummer: PCT/EP2008/010426
(87) Internationale Veröffentlichungsnummer: WO 2009/095057

(56) Entgegenhaltungen:
- EP-A1- 0 870 498
- WO-A1-2004/047816
- DE-A1- 4 210 165
- FR-A1- 2 867 978
- US-A- 3 981 996
- US-A- 4 687 481
- US-A- 4 699 777
- US-A- 5 230 896
- VALENTA C ET AL: "Effect of urea and pantothenol on the permeation of progesterone through excised rat skin from polymer matrix systems" DRUG DEVELOPMENT AND INDUSTRIAL PHARMACY, NEW YORK, NY, US, Bd. 27, 1. Januar 2001 (2001-01-01), Seiten 57-62, XP008115116 ISSN: 0363-9045
- VALENTA C ET AL: "In vitro release study of transdermal delivery systems of progesterone" DRUG DEVELOPMENT AND INDUSTRIAL PHARMACY, NEW YORK, NY, US, Bd. 24, Nr. 2, 1. Januar 1998 (1998-01-01) , Seiten 187-191, XP008115104 ISSN: 0363-9045
- DATABASE CA CHEMICAL ABSTRACTS SERVICE, COLUMBUS, OHIO, US; 23. Dezember 1997 (1997-12-23), XP002561598 gefunden im stn Database accession no. 127:362624 & WO 97/39740 A1 (TOYAMA CHEMICAL CO LTD [JP]; YAMAKAWA TETSUMI [JP]; TAKAKURA KEIKO [JP) 30. Oktober 1997 (1997-10-30)
- "There's more than one way to measure powder particle size!", METAL POWDER REPORT, MPR PUBLISHING SERVICES, SHREWSBURY, GB, vol. 58, no. 11, 11 October 2003 (2003-10-11), pages 26-31, XP004471292, ISSN: 0026-0657
- Remi Trottier ET AL: "Particle Size Measurement", Kirk-Othmer Encyclopedia of Chemical Technology, 14 October 2005 (2005-10-14), XP055005783, DOI: 10.1002/0471238961.190926052018152 Retrieved from the Internet: URL:http://onlinelibrary.wiley.com/store/1 0.1002/0471238961.1909260520181520.a01.pub 2/asset/sizetrot.a01.pdf?v=1&t=grymnd2r&s= 6970b9c8fceb00b1ecc738aefe0f8765b2377ee5 [retrieved on 2011-08-30]

## Beschreibung

Transdermale Therapeutische Systeme (TTS) sind pharmazeutische Darreichungsformen, die auf die Haut eines Säugers appliziert werden, und dafür konzipiert sind, einen Arzneistoff nach transdermaler Aufnahme systemisch verfügbar zu machen. TTS können den therapeutischen Wert der Verabreichung eines Arzneistoffes erhöhen, indem sie eine konstante Abgabe des Wirkstoffs über einen längeren Zeitraum in das Blutkompartiment gewährleisten. Die Vorteile dieser kontinuierlichen Wirkstoffabgabe sind in erster Linie die verlängerten Applikationsintervalle, die zu einer verbesserten Patientenkompliance führen und das pharmakokinetisch optimierte Plasmakonzentrations-Zeitprofil, das eine längere Wirkdauer bei weniger Nebenwirkungen gewährleistet. Weitere Vorteile, die in der transdermalen Applikationsroute mittels eines TTS begründet liegen, sind geringere Dosierung, verbesserte gastrointestinale Verträglichkeit und eine verbesserte Bioverfügbarkeit durch Umgehung des so genannten "First-Pass-Effektes".

Aufgrund dieser Vorteile erfreuen sich TTS seit einer Reihe von Jahren einer wachsenden Beliebtheit bei der Therapie diverser Erkrankungen. Solche Systeme sind zum Beispiel für die Wirkstoffe Estradiol, Nikotin, Norethisteronacetat, Fentanyl, Tulobuterol, Ethinylestradiol, Buprenorphin und Nitroglycerin in die Therapie eingeführt. Der Aufbau eines TTS ist in der Regel dünn und geschichtet, so dass sich mit Hilfe der direkt der Haut zugewandten Schicht (H) eine zumindest vorübergehende klebrige Verbindung zur Haut ergibt, über die der Wirkstoff abgegeben wird. TTS bestehen üblicherweise aus einer für den Arzneistoff undurchlässigen Rückschicht (R), der so genannten "Backing Layer", einer wirkstoffhaltigen Schicht (S), beispielsweise einer Reservoirschicht oder Matrixschicht sowie einer Klebschicht (K) zur Befestigung auf der Haut, wobei diese mit der arzneistoff- bzw. wirkstoffhaltigen Schicht (z. B. Reservoirschicht oder Matrixschicht) identisch sein kann, und einer vor der Applikation zu entfernenden Arzneistoff undurchlässigen Schutzschicht (A), dem so genannten "Release Liner".

Zur Verbesserung der Permeation des jeweiligen Wirkstoffes durch die Haut werden neben verschiedenen festen Polymeren (z.B. Polyacrylate, Silicone, Polyisobutylene), Harzen und anderen pharmazeutischen Hilfsstoffen auch verschiedene, bei Raumtemperatur flüssige Systemkomponenten eingesetzt, die zum Teil eine Einstellung der Klebkraft ermöglichen und der Diffusionsverbesserung innerhalb des transdermalen therapeutischen Systems oder aber der Verbesserung der Wirkstoffpermeation durch die Haut dienen.

Zahlreiche der bekannten Wirkstoffe eignen sich für eine Applikation über die Haut, z.B. weil sie aufgrund ihres geringen Molekulargewichts und/oder ihrer hohen Lipophile bereits ohne weitere Hilfsmaßnahmen die menschliche Haut passieren können. Beispiele hierfür sind die Wirkstoffe Nikotin, Nitroglycerin, Steroidhormone und Clonidine. Für viele pharmazeutische Wirkstoffe ist allerdings bislang die Applikation über die transdermale Route verschlossen, weil ihre Tagesdosierung zu hoch ist, um über eine vernünftige Hautfläche applizierbar zu sein.

Es wurden bereits zahlreiche technische Lösungen, wie der Zusatz von permeationsfördernden Stoffen, die Anwendung von elektrischer Spannung (Iontophorese) oder Ultraschall, und auch von Mikroläsionen der Haut vorgeschlagen und zumindest zum Teil auch experimentell erfolgreich geprüft. Es gibt mehrere Möglichkeiten, den Wirkstofffluss durch die Haut zu erhöhen. In der Regel gehen diese Maßnahmen jedoch mit einer Einschränkung der Hautverträglichkeit einher, so dass eine Risikoabwägung des Arztes erfolgen muss, die dann in der Regel zugunsten einer klassischen Applikationsform ausfällt.

Eine Aufgabe der vorliegenden Erfindung ist die Bereitstellung eines transdermalen therapeutischen Systems, welches den Fluss des Wirkstoffes durch die Haut deutlich verstärkt und gleichzeitig eine gute (oder mindestens akzeptable) Hautverträglichkeit aufweist.

Die Lösung der erfinderischen Aufgabe erfolgt durch Zusatz des in der Haut des Säugers (z.B. Menschen) ohnehin bereits in geringen Mengen vorkommenden Hilfsstoffes Harnstoff in fester Form.

Der Einsatz von Harnstoff in allgemeiner Form als Permeationsförderer ist nicht grundsätzlich unbekannt. Ein förderlicher Einfluss von Harnstoff auf die Hautpermeation wird z.B. von W. Wohlrab (Acta Derm. Venerol 1984, 64, 233 - 238) beschrieben, wobei hier eine Formulierung von Hydrocortison als Emulsion mit Harnstoff vorgestellt wird.

Von C. K. Kim (Intern. J. of Pharmaceutics 1993, 99, 109 - 118) wird der Einfluss von Harnstoff-Lösungen auf die Penetration von Ketoprofen durch die Mäusehaut beschrieben.

In der Veröffentlichung von V.L.B. Bentley (Intern. J. of Pharmaceutics 1997, 146, 255 bis 262) wird die Permeationssteigerung für Hydrocortison durch Harnstoff-haltige Gele offenbart. Der Einfluss von Harnstoff auf die menschliche Haut wird auch von P. Clarys (Skin Pharmacology and Applied Skin Physiology 1999, 12, 85 - 89) beschrieben.

US4699777 offenbart topische Zusammensetzungen, bevorzugt in Form eines transdermalen Pflasters, enthaltend den Wirkstoff Albuterol und Harnstoff als Penetrationsverstärker in einer Konzentration von 5-50%.

Es sind jedoch bislang keine marktüblichen Systeme bekannt, in denen ein hoher Anteil von festem, als grobkörnige Partikel vorliegendem Harnstoff eingesetzt wird.

Gegenstand der vorliegenden Erfindung ist ein transdermales therapeutisches System (TTS) zur Abgabe pharmazeutischer Wirkstoffe durch die Haut, enthaltend eine Wirkstoffundurchlässige Rückschicht (R) und mindestens eine Wirkstoff-haltige Schicht (S), bei dem die der Haut zugewandte Schicht (H) festen Harnstoff enthält entsprechend der Ansprüche.

Im TTS beträgt der Gewichtsanteil (genauer Wasseranteil) des Harnstoffes am Grundmaterial der der Haut zugewandten Schicht (H) des TTS mindestens 20 % (m/m).

Die Erfindung betrifft ferner ein TTS, bei dem der in der Schicht (H) enthaltene Harnstoff im Wesentlichen in fester, grob-kristalliner Form enthalten ist.

Die Erfindung betrifft auch ein TTS, bei dem die Wirkstoff-haltige Schicht (S) zugleich die der Haut zugewandte Schicht (H) ist, und diese Schicht neben 1 bis 20 % (m/m), insbesondere 1 bis 15 % (m/m), mindestens eines pharmazeutischen Wirkstoffs auch 20 bis 50 % (m/m) an Harnstoff enthält.

Im TTS liegt der in der Schicht (H) enthaltene Harnstoff zu mindestens 50 Gew.-% in einer Korngröße von über 50 µm, vorzugsweise über 70µ und insbesondere über 100 µm vor. Die Korngröße bzw. Korngrößenverteilung kann beispielsweise durch den Einsatz von Sieben gemessen werden.

Die Erfindung betrifft auch ein TTS, bei dem der in der Schicht (H) enthaltene Harnstoff zu mindestens 70 Gew.-% in einer Korngröße von über 70 µm vorliegt.

Die Erfindung betrifft auch ein TTS, bei dem der in der Schicht (H) enthaltene kristalline Harnstoff zu mindestens 70 Gew.-% in einer Korngröße von über 100 µm vorliegt.

Die Erfindung betrifft auch ein TTS, bei dem es sich bei der Wirkstoff-haltigen Schicht (S) um eine Polymer-Matrix, insbesondere eine Polyacrylatmatrix, handelt, die neben 2 bis 18 % (m/m) mindestens eines pharmazeutischen Wirkstoffs auch 20 bis 40 % (m/m) an Hartstoff enthält.

Gegenstand der Erfindung ist auch ein TTS, wobei es sich bei der Wirkstoff-haltigen Schicht (S) um eine Polymer-Matrix auf Basis eines Polyacrylats und/oder eines Polymethacrylats handelt, die neben 5 bis 18 % (m/m) mindestens eines pharmazeutischen Wirkstoffs auch 20 bis 60 % (m/m) an Harnstoff enthält, der zu mindestens 50 Gew.-% in einer Korngröße von über 50 µm, vorzugsweise über 70µ und insbesondere über 100 µm, vorliegt.

Die Erfindung betrifft auch ein TTS, bei dem es sich bei der Wirkstoff-haltigen Schicht (S) um eine Polymer-Matrix auf Basis eines Polyacrylats und/oder eines Polymethacrylats handelt, die neben einem pharmazeutischen Wirkstoff aus der Gruppe der Muskelrelaxansien, Antihypertonika, Psychostimulatien und Antiemetika auch 20 bis 40 % (m/m) an kristallinem Harnstoff enthält, der zu mindestens 70 Gew.-% in einer Korngröße von über 70 µm (und insbesondere über 100 µm) vorliegt.

Die Erfindung betrifft auch Verfahren zur Herstellung eines transdermalen therapeutischen Systems entsprechend der Ansprüche, bei dem man auf eine Wirkstoff undurchlässige Rückschicht (R) mindestens eine Wirkstoff-haltige Schicht (S) und ggf. weitere Schichten aufbringt, wobei die der Haut zugewandte Schicht (H) Harnstoff in fester, vorzugsweise kristalliner, Form enthält.

Ein weiterer Aspekt ist die Verwendung einer Wirkstoff-haltigen Polymerschicht (S), die zusätzlich festen Harnstoff enthält, zur Herstellung eines transdermalen therapeutischen Systems entsprechend Anspruch 1 zur Behandlung von Erkrankungen bei Mensch und Tier entsprechend Anspruch 10.

Aufgrund der nachstehenden Versuchsergebnisse erwies es sich als überraschend, dass anders als bei gelöstem oder feinteiligen Harnstoff, der Zusatz von festem, als grobkörnigen Partikel vorliegenden und anteilsmäßig mindestens 20 % liegenden Harnstoff eine deutliche, größenordnungsmäßig sehr relevante Permeationsförderungen bewirkt.

Der die Permeation fördernde Effekt des festen, als grobkörnige Partikel vorliegenden Harnstoffs konnte dabei für unterschiedliche Wirkstoff-Gruppen wie beispielsweise Muskelrelaxansien (Tizanidin), Antihypertonika (Moxonidin), Psychostimulatien (Coffein) und Antiemetika (Lerisetron) nachgewiesen werden.

Der Aufbau eines TTS ist dabei vorzugsweise mehrschichtig und enthält mindestens eine wirkstoffhaltige Schicht (S) und eine Klebschicht, wobei die wirkstoffhaltige Schicht auch zugleich die Klebschicht sein kann. Besonders geeignet erwies sich ein TTS, bei dem die Klebschicht des Systems einen Harnstoff-Anteil von mindestens 20 % (m/m) aufweist. Vorzugsweise liegt dabei der enthaltene Harnstoff zu mindestens 80% in fester Form als grobkörnige Partikel vor. Die festen grobkörnigen Partikel wiederum haben vorzugsweise eine Korngröße von mindestens 50 µm, besser über 70µm und insbesondere von über 100 µm. Bei dem eingesetzten Harnstoff kann es sich vorzugsweise um kristallinen Harnstoff handeln.

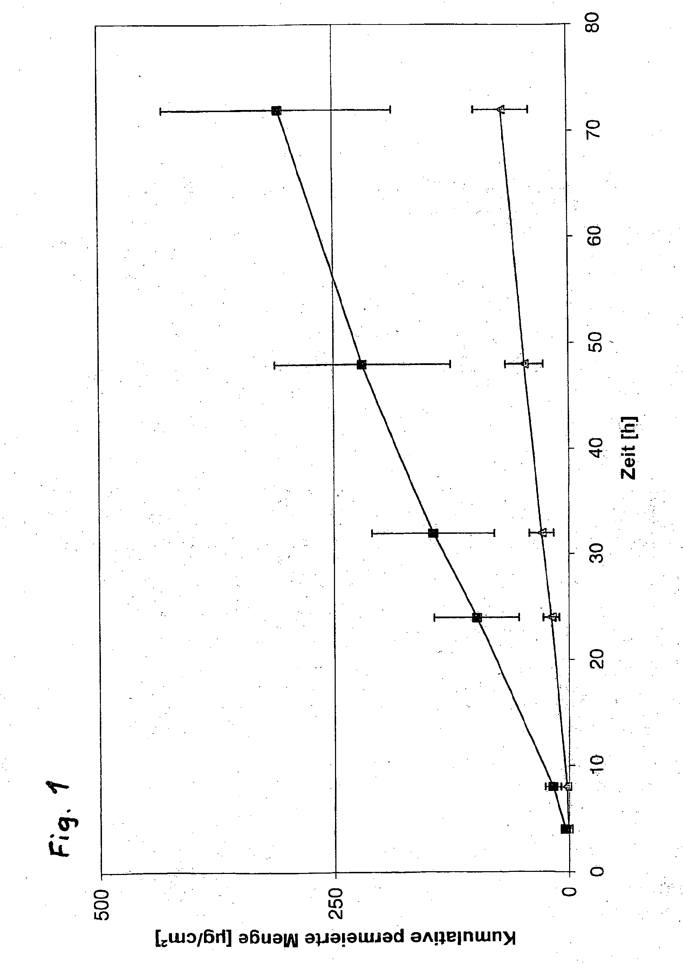

Die Erfindung wird durch die nachstehenden Beispiele näher erläutert. Die Abbildungen (Fig. 1 bis 4) zeigen die kulumative permeierte Menge des Wirkstoffs (in µg pro cm²) als Ordinate und die Zeit (in Stunden) als Abszisse. Die mit kleinen Dreiecken gekennzeichneten Kurven in den Figuren 1, 2 und 4 zeigen die Ergebnisse bei TTS ohne Harnstoff-Zusatz, die mit kleinen Quadraten gekennzeichneten Kurven zeigen die Ergebnisse mit 20 % Harnstoff-Zusatz (wobei die Korngröße 90 bis 125 µm betrug). Man erkennt dabei eine deutliche Permeationssteigerung um den Faktor 4 bis 9 für verschiedene Wirkstoffe durch Zugabe von festem Harnstoff.

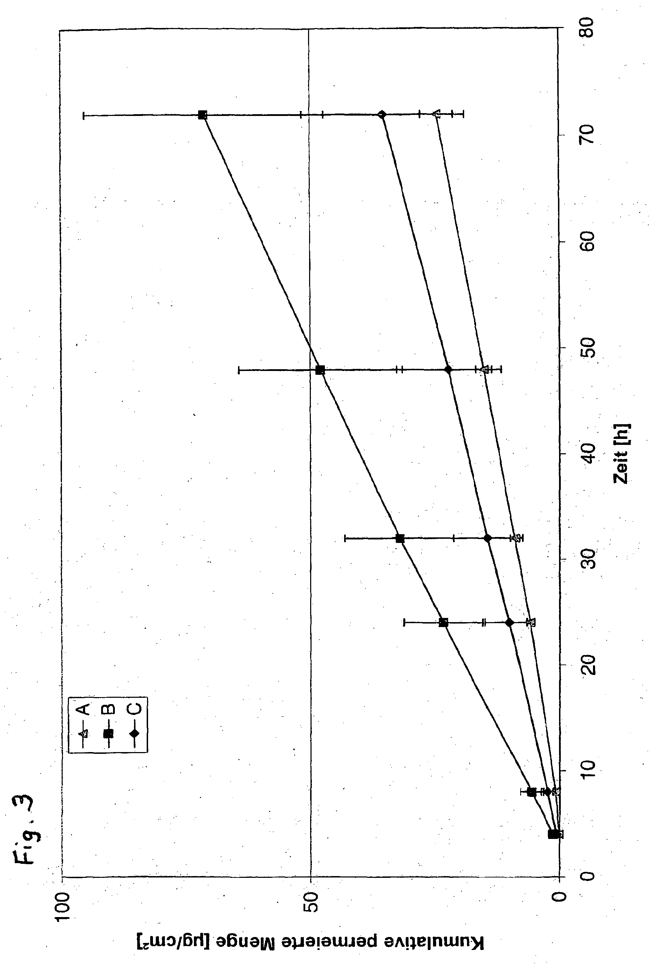

Fig 3. zeigt eine unerwartet deutliche Erhöhung der Wirkstoffpermeation des TTS mit Harnstoff in einer Partikelgröße > 100 µm (Kurve B) gegenüber dem Vergleichsbeispiel eines TTS mit Harnstoff mit einer Partikelgröße < 50µm (Kurve C). Die mit kleinen Dreiecken gekennzeichneten Kurve A in Figur 3 zeigt die Ergebnisse bei TTS ohne Harnstoff-Zusatz.

Beispiel 1 Aufbau eines Matrixsystems-TTS:
- Abziehbare Schutzschicht (silikonbeschichtete PET-Folie)
- Klebschicht: hydrophiler Acrylatklebstoff (zum Beispiel Durotak® 387-2287, mit 10 % (m/m) Tizanidin und 20 % (m/m) Harnstoff, wobei der Harnstoff in fester Form als grobkörnige Partikel mit einer Partikelgröße > 100 µm vorliegt.
- Abdeckfolie (PET-Folie)

Ein handelsüblicher Acrylat-Klebstoff wurde in einem Lösungsmittel aufgelöst. Der Acrylat-Klebstofflösung wurden der Wirkstoff Tizanidin und fester, als grobkörnige Partikel vorliegender Harnstoff in den oben genannten Mengen unter Rühren zugesetzt. Diese Acrylat-Klebstoffmasse wurde zu einer Vorratsschicht von 500 µm Dicke gegossen und das Lösungsmittel abgedampft, so dass ein Matrix-Flächengewicht von 100 g/m² resultiert. Aus diesem Wirkstoff-haltigen Laminat wurden mehrere Versuchs-TTS ausgestanzt, die dann für in vitro Versuche verwendet wurden.

Die in vitro Permeationsversuche wurden in einer im Stand der Technik beschriebenen Franz-Diffusionszelle durchgeführt. Die Franz-Diffusionszelle besteht aus einem Donorund einem Akzeptorkompartiment, die durch eine Membran (Kuheuter) getrennt sind. Das Donorkompartiment enthält das TTS und als Akzeptorkompartiment wurde ein physiologischer Puffer verwendet, der auf 32 °C temperiert ist. Aus dem Akzeptorkompartiment wurden über einen Zeitraum von 72 h Proben entnommen, von denen mittels HPLC die permeierte Wirkstoffmenge bestimmt wurde.

Die Messergebnisse sind in Figur 1 als Permeationsprofil des Wirkstoffs durch Kuheuter abgebildet. Die kumulierte permeierte Wirkstoffmenge (Mikrogramm pro Quadratzentimeter) aus einem TTS, dass keinen Harnstoff enthält (A) und einem TTS, dass Harnstoff in einer Partikelgröße > 100µm enthält (B) wurde gegen die Zeit aufgetragen. Man erkennt die deutliche Erhöhung der Tizanidinpermeation durch die Haut um den Faktor 4.

Beispiel 2 Aufbau eines Matrixsystem-TTS:
- Abziehbare Schutzschicht (silikonbeschichtete PET-Folie)
- Klebschicht: hydrophiles Acrylat (zum Beispiel Durotak 387-2287) mit 10 % (m/m) Coffein und 20 % (m/m) Harnstoff, wobei der Harnstoff kristallin mit einer Partikelgröße > 100 µm vorliegt.
- Wirkstofffreie Schicht (36g/m²): hydrophobes Polymer mit einem Harz abgemischt (zum Beispiel Kraton®/ Foral; 1/4)
- Abdeckfolie (PET-Folie)

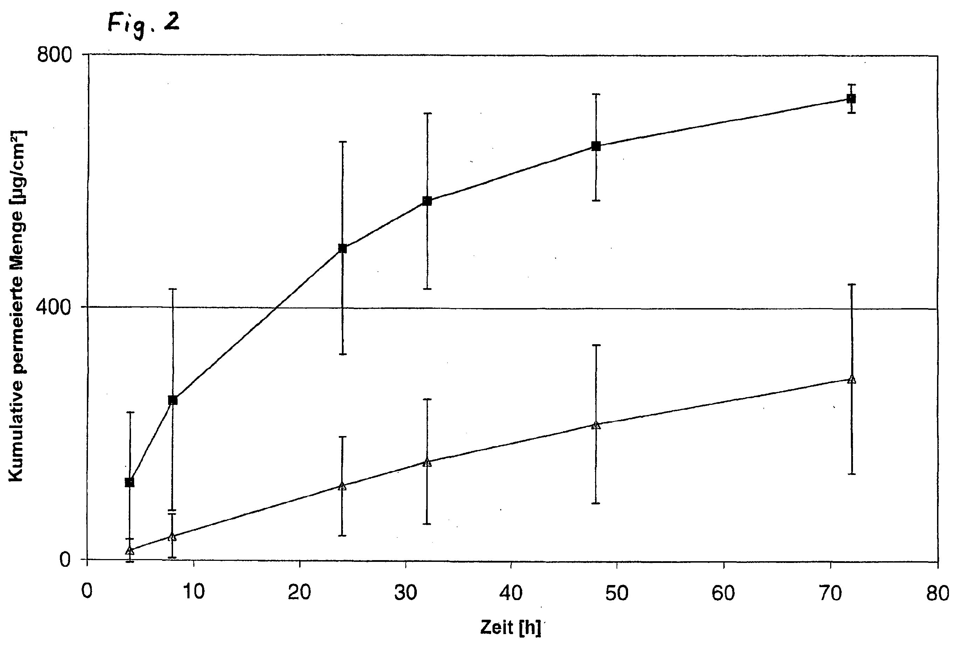

Die TTS wurde wie in Beispiel 1 beschrieben hergestellt und untersucht. Die kumulierte, permeierte Wirkstoffmenge (Mikrogramm pro Quadratzentimeter) aus einem TTS, das keinen Harnstoff enthält (A), und einem TTS, das Harnstoff in einer Partikelgröße > 100µm enthält (B), wurde gegen die Zeit aufgetragen (Figur 2). Man erkennt die deutliche Erhöhung der Coffein-Permeation durch die Haut um den Faktor 8.

Beispiel 3 Aufbau eines Matrixsystem-TTS:
- Abziehbare Schutzschicht (silikonbeschichtete PET-Folie)
- Klebschicht: hydrophiles Acrylat (zum Beispiel Durotak® 387-2287) mit 10 % (m/m) Moxonidin und 20 % (m/m) Harnstoff, wobei der Harnstoff kristallin mit einer Partikelgröße > 100 µm vorliegt.
- Abdeckfolie (PET-Folie)

Die TTS wurde wie in Beispiel 1 beschrieben hergestellt und untersucht. Die kumulierte permeierte Wirkstoffmenge (Mikrogramm pro Quadratzentimeter) aus einem TTS, das keinen Harnstoff enthält (A), einem TTS, dass 10% Harnstoff mit einer Partikelgröße < 50µm (C) und einem TTS, das 20 % Harnstoff in einer Partikelgröße > 100µm enthält (B), wurde gegen die Zeit aufgetragen (Figur 3). Man erkennt die deutliche Erhöhung der Moxonidin-Permeation durch den 20%igen Harnstoffanteil mit einer Partikelgröße > 100µm.

Beispiel 4 Aufbau eines Matrixsystem-TTS:
- Abziehbare Schutzschicht (silikonbeschichtete PET-Folie)
- Klebschicht: hydrophiles Acrylat (zum Beispiel Durotak® 387-2287) mit 10 % (m/m) Lerisetron und 20 % (m/m) Harnstoff, wobei der Harnstoff kristallin mit einer Partikelgröße > 100 µm vorliegt.
- Abdeckfolie (PBT=Folie)

Die TTS wurde wie in Beispiel 1 beschrieben hergestellt und untersucht.

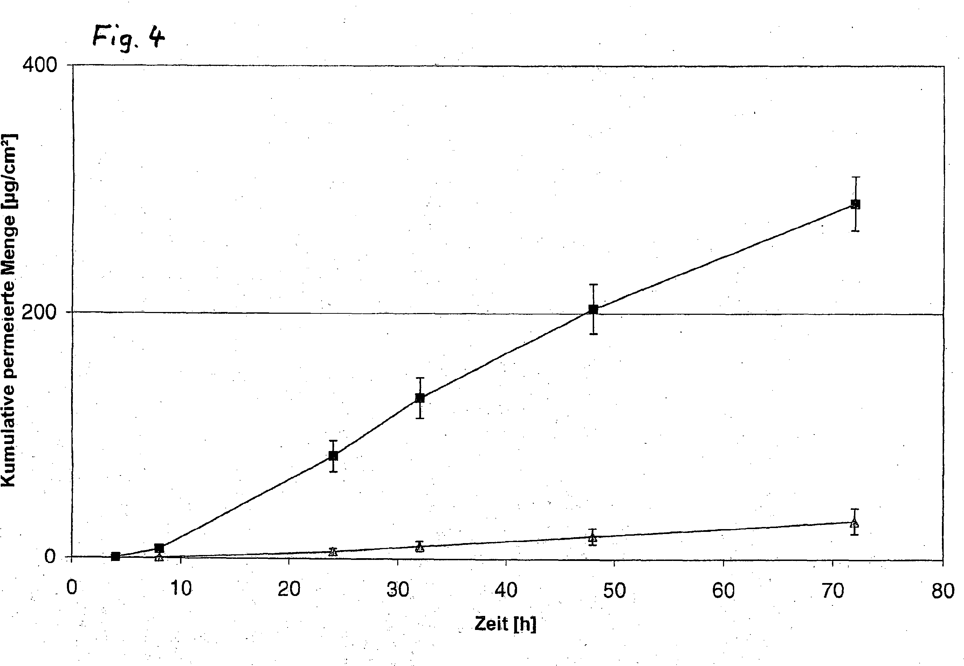

Die kumulierte permeierte Menge an Lerisetron wurde gegen die Zeit aufgetragen und ist in Figur 4 dargestellt. Die kumulierte permeierte Wirkstoffmenge (Mikrogramm pro Quadratzentimeter) aus einem TTS, das keinen Harnstoff enthält (A), und einem TTS, das Harnstoff in einer Partikelgröße > 100µm enthält (B), wurde gegen die Zeit aufgetragen (Figur 4). Man erkennt die deutliche Erhöhung der Lerisetronpermeation durch die Haut um den Faktor 9.

## Patentansprüche

1. Transdermales therapeutisches System (TTS) zur Abgabe pharmazeutischer Wirkstoffe durch die Haut, enthaltend eine Wirkstoff-undurchlässige Rückschicht (R) und mindestens eine Wirkstoff-haltige Schicht (S), bei dem die der Haut zugewandte Schicht (H) festen Harnstoff enthält, wobei der Gewichtsanteil des Harnstoffes am Grundmaterial der Schicht (H) mindestens 20 % (m/m) beträgt und der enthaltene Harnstoff zu mindestens 50 Gew.-% in einer Korngröße von über 50 µm vorliegt.

2. Transdermales therapeutisches System nach Anspruch 1, **dadurch gekennzeichnet, dass** der in der Schicht (H) enthaltene Harnstoff im Wesentlichen in fester, grob-kristalliner Form enthalten ist.

3. Transdermales therapeutisches System nach einem der Ansprüche 1 bis 2, **dadurch gekennzeichnet, dass** die Wirkstoff-haltige Schicht (S) zugleich die der Haut zugewandte Schicht (H) ist, und diese Schicht neben 1 bis 20 % (m/m) mindestens eines pharmazeutischen Wirkstoffs auch 20 bis 50 % (m/m) an Harnstoff enthält.

4. Transdermales therapeutisches System nach einem der Ansprüche 1 bis 3, **dadurch gekennzeichnet, dass** der in der Schicht (H) enthaltene Harnstoff zu mindestens 50 Gew.-% in einer Korngröße von über 70 µm vorliegt.

5. Transdermales therapeutisches System nach einem der Ansprüche 1 bis 4, **dadurch gekennzeichnet, dass** der in der Schicht (H) enthaltene Harnstoff zu mindestens 50 Gew.-% in einer Korngröße von über 100 µm vorliegt.

6. Transdermales therapeutisches System nach einem der Ansprüche 1 bis 5, **dadurch gekennzeichnet, dass** es sich bei der Wirkstoff-haltigen Schicht (S) um eine Polymer-Matrix handelt, die neben 2 bis 18 % (m/m) mindestens eines pharmazeutischen Wirkstoffs auch 20 bis 40% (m/m) an Harnstoff enthält.

7. Transdermales therapeutisches System nach einem der Ansprüche 1 bis 6, **dadurch gekennzeichnet, dass** es sich bei der Wirkstoff-haltigen Schicht (S) um eine Polymer-Matrix auf Basis eines Polyacrylats und/oder eines Polymethacrylats handelt, die neben 5 bis 18 % (m/m) mindestens eines pharmazeutischen Wirkstoffs auch 20 bis 60 % (m/m) an Harnstoff enthält, der zu mindestens 50 Gew -% in einer Korngröße von über 50 µm vorliegt.

8. Transdermales therapeutisches System nach einem der Ansprüche 1 bis 7, **dadurch gekennzeichnet, dass** es sich bei der Wirkstoff-haltigen Schicht (S) um eine Polymer-Matrix auf Basis eines Polyacrylats und/oder eines Polymethacrylats handelt, die neben einem pharmazeutischen Wirkstoff aus der Gruppe der Muskelralaxantien, Antihypertonika, Psychostimulantien und Antiemetika auch 20 bis 40 % (m/m) an kristallinem Harnstoff enthält, der zu mindestens 70 Gew.-% in einer Korngröße von über 70 µm vorliegt.

9. Verfahren zur Herstellung eines transdermalen therapeutischen Systems gemäß einem der Ansprüche 1 bis 8, **dadurch gekennzeichnet, dass** man auf eine Wirkstoff-undurchlässige Rückschicht (R) mindestens eine Wirkstoff-haltige Schicht (S) und ggf. weitere Schichten aufbringt, wobei die der Haut zugewandte Schicht (H) Harnstoff in fester Form enthält.

10. Verwendung einer Wirkstoff-haltigen Polymerschicht (S), die zusätzlich festen Harnstoff enthält, zur Herstellung eines transdermalen therapeutischen Systems nach Anspruch 1 zur Behandlung von Erkrankungen bei Mensch und Tier, wobei der Gewichtsanteil des Harnstoffes mindestens 20 % (m/m) beträgt und wobei der enthaltene Harnstoff zu mindestens 50 Gew.-% in einer Korngröße von über 50 µm vorliegt.

## Claims

1. Transdermal therapeutic system (TTS) for delivering active pharmaceutical ingredients through the skin, comprising an ingredient-impermeable backing layer (R) and at least one ingredient-containing layer (S), wherein the skin-facing layer (H) comprises solid urea, the weight fraction of the urea as a proportion of the base material of the layer (H) being at least 20% (m/m) and the urea present being present to an extent of at least 50% by weight in a particle size of more than 50 µm.

2. Transdermal therapeutic system according to Claim 1, **characterized in that** the urea present in layer (H) is present substantially in solid, coarsely crystalline form.

3. Transdermal therapeutic system according to one of Claims 1 or 2, **characterized in that** the ingredient-containing layer (S) is also the skin-facing layer (H), and this layer, in addition to 1% to 20% (m/m) of at least one active pharmaceutical ingredient, comprises 20% to 50% (m/m) of urea.

4. Transdermal therapeutic system according to any of Claims 1 to 3, **characterized in that** the urea present in layer (H) is present to an extent of at least 50% by weight in a particle size of more than 70 µm.

5. Transdermal therapeutic system according to any of Claims 1 to 4, **characterized in that** the urea present in layer (H) is present to an extent of at least 50% by weight in a particle size of more than 100 µm.

6. Transdermal therapeutic system according to any of Claims 1 to 5, **characterized in that** the ingredient-containing layer (S) is a polymer matrix which, in addition to 2% to 18% (m/m) of at least one active pharmaceutical ingredient, comprises 20% to 40% (m/m) of urea.

7. Transdermal therapeutic system according to any of Claims 1 to 6, **characterized in that** the ingredient-containing layer (S) is a polymer matrix based on a polyacrylate and/or a polymethacrylate, and in addition to 5% to 18% (m/m) of at least one active pharmaceutical ingredient, comprises 20% to 60% (m/m) of urea, which is present to an extent of at least 50% by weight in a particle size of more than 50 µm.

8. Transdermal therapeutic system according to any of Claims 1 to 7, **characterized in that** the ingredient-containing layer (S) is a polymer matrix based on a polyacrylate and/or a polymethacrylate, and in addition to an active pharmaceutical ingredient from the group consisting of muscle relaxants, antihypertensives, psychostimulants, and antiemetics, comprises 20% to 40% (m/m) of crystalline urea, which is present to an extent of at least 70% by weight in a particle size of more than 70 µm.

9. Method of producing a transdermal therapeutic system according to any of Claims 1 to 8, **characterized in that** at least one ingredient-containing layer (S) and, if desired, further layers are applied to an ingredient-impermeable backing layer (R), the skin-facing layer (H) comprising urea in solid form.

10. Use of an active-ingredient-containing polymer layer (S) further comprising solid urea for producing a transdermal therapeutic system according to Claim 1 for treating illnesses in humans and animals, the weight fraction of the urea being at least 20% (m/m) and the urea present being present to an extent of at least 50% by weight in a particle size of more than 50 µm.

## Revendications

1. Système thérapeutique transdermique (STT) destiné à l'administration de substances actives pharmaceutiques à travers la peau, contenant une couche de dos (R) imperméable aux substances actives et au moins une couche (S) contenant une substance active, dans lequel la couche (H) tournée vers la peau contient de l'urée solide, la proportion pondérale de l'urée par rapport au matériau de base de la couche (H) valant au moins 20 % (m/m) et l'urée contenue se trouvant à raison d'au moins 50 % en poids en une taille de grain de plus de 50 µm.

2. Système thérapeutique transdermique selon la revendication 1, **caractérisé en ce que** l'urée contenue dans la couche (H) est essentiellement contenue sous forme solide en gros cristaux.

3. Système thérapeutique transdermique selon l'une quelconque des revendications 1 et 2, **caractérisé en ce que** la couche (S) contenant une substance active est en même temps la couche (H) tournée vers la peau, et cette couche contient outre 1 à 20 % (m/m) d'au moins une substance active pharmaceutique également 20 à 50 % (m/m) d'urée.

4. Système thérapeutique transdermique selon l'une quelconque des revendications 1 à 3, **caractérisé en ce que** l'urée contenue dans la couche (H) est présente à raison d'au moins 50 % en poids en une taille de grain de plus de 70 µm.

5. Système thérapeutique transdermique selon l'une quelconque des revendications 1 à 4, **caractérisé en ce que** l'urée contenue dans la couche (H) est présente à raison d'au moins 50 % en poids en une taille de grain de plus de 100 µm.

6. Système thérapeutique transdermique selon l'une quelconque des revendications 1 à 5, **caractérisé en ce que** pour ce qui est de la couche (S) contenant une substance active il s'agit d'une matrice polymère qui outre 2 à 18% (m/m) d'au moins une substance active pharmaceutique contient également 20 à 40% (m/m) d'urée.

7. Système thérapeutique transdermique selon l'une quelconque des revendications 1 à 6, **caractérisé en ce que** pour ce qui est de la couche (S) contenant une substance active il s'agit d'une matrice polymère à base d'un polyacrylate et/ou d'un polyméthacrylate qui outre 5 à 18% (m/m) d'au moins une substance active pharmaceutique contient également 20 à 60 % (m/m) d'urée qui est présente à raison d'au moins 50 % en poids en une taille de grain de plus de 50 µm.

8. Système thérapeutique transdermique selon l'une quelconque des revendications 1 à 7, **caractérisé en ce que** pour ce qui est de la couche (S) contenant une substance active il s'agit d'une matrice polymère à base d'un polyacrylate et/ou d'un polyméthacrylate qui outre une substance active pharmaceutique choisie dans le groupe des myorelaxants, antihypertoniques, psychostimulants et antiémétiques contient également 20 à 40 % (m/m) d'urée cristalline qui est présente à raison d'au moins 70 % en poids en une taille de grain de plus de 70 µm.

9. Procédé pour la fabrication d'un système thérapeutique transdermique selon l'une quelconque des revendications 1 à 8, **caractérisé en ce qu'**on applique sur une couche de dos (R) imperméable aux substances actives au moins une couche (S) contenant une substance active et éventuellement d'autres couches, la couche (H) tournée vers la peau contenant de l'urée sous forme solide.

10. Utilisation d'une couche de polymère (5) contenant une substance active, qui contient en outre de l'urée, pour la fabrication d'un système thérapeutique transdermique selon la revendication 1, destiné au traitement de maladies chez l'homme et l'animal, la proportion en poids de l'urée valant au moins 20 %(m/m) et l'urée contenue étant présente à raison d'au moins 50 % en poids en une taille de grain de plus de 50 µm.
